(19) **Europäisches Patentamt**
**European Patent Office**
**Office européen des brevets**

(11) **EP 2 158 484 B1**

(12) **FASCICULE DE BREVET EUROPEEN**

(45) Date de publication et mention
de la délivrance du brevet:
**02.11.2011 Bulletin 2011/44**

(51) Int Cl.:
*G01N 33/06* (2006.01)       *A23K 1/18* (2006.01)

(21) Numéro de dépôt: **09756121.1**

(22) Date de dépôt: **24.06.2009**

(86) Numéro de dépôt international:
**PCT/EP2009/057919**

(87) Numéro de publication internationale:
**WO 2009/156453 (30.12.2009 Gazette 2009/53)**

(54) **PROCÉDÉ D'ÉVALUATION DE LA QUANTITÉ DE MÉTHANE PRODUITE PAR UN RUMINANT LAITIER ET PROCÉDÉ POUR DIMINUER ET CONTRÔLER CETTE QUANTITÉ**

VERFAHREN ZUM AUSWERTEN DER VON EINEM MILCHPRODUZIERENDEN WIEDERKÄUER ERZEUGTEN METHANMENGE, UND VERFAHREN ZUR ERNIEDRIGUNG UND KONTROLLE DIESER MENGE

METHOD FOR EVALUATING THE AMOUNT OF METHANE PRODUCED BY A DAIRY RUMINANT AND METHOD FOR DECREASING AND CONTROLLING THIS AMOUNT

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **25.06.2008 FR 0854230**

(43) Date de publication de la demande:
**03.03.2010 Bulletin 2010/09**

(73) Titulaire: **Valorex**
**35210 Combourtille (FR)**

(72) Inventeurs:
• **WEILL, Pierre**
**F-35770 Vern Sur Seiche (FR)**
• **CHESNEAU, Guillaume**
**F-35133 Luitre (FR)**
• **CHILLIARD, Yves**
**F-63122 Ceyrat (FR)**
• **DOREAU, Michel**
**F-63450 Saint Saturnin (FR)**
• **MARTIN, Cécile**
**F-63450 Saint Saturnin (FR)**

(74) Mandataire: **Branger, Jean-Yves et al**
**Cabinet Regimbeau**
**Espace Performance**
**Bâtiment K**
**35769 Saint-Gregoire-Cedex (FR)**

(56) Documents cités:
**WO-A-2006/040537       WO-A-2007/141416**

• **ELLIS J. ET AL.: "prediction of methane production from dairy and beef cattle" JOURNAL OF DAIRY SCIENCE, 2007, pages 3456-3467, XP002509496**
• **SOYEURT H ET AL: "Estimating fatty acid content in cow milk using mid-infrared spectrometry" JOURNAL OF DAIRY SCIENCE, AMERICAN DAIRY SCIENCE ASSOCIATION, SAVOY, IL, US, vol. 89, no. 9, 1 septembre 2006 (2006-09-01), pages 3690-3695, XP002455357 ISSN: 0022-0302**
• **B. VLAEMINCK, V. FIEVEZ: "Milk odd and branched-chain fatty acids to predict ruminal methanogenesis in dairy cows", COMMUNICATIONS IN AGRICULTURAL AND APPLIED BIOLOGICAL SCIENCES, vol. 70, no. 2, 2005, pages 43-47,**

**Description**

La présente invention se situe dans le domaine de l'alimentation animale et, plus précisément, le domaine de l'alimentation des animaux destinés à la production de lait.

Elle concerne plus particulièrement un procédé d'évaluation de la quantité de méthane produite par un ruminant laitier, ainsi qu'un procédé pour contrôler la quantité de méthane produite par un ruminant laitier.

La digestion de la matière organique par les ruminants comporte une phase de fermentation microbienne dans le rumen. Au cours de celle-ci, les polyosides végétaux (tels que la cellulose, les hémicelluloses, les pectines et l'amidon) sont dégradés par les bactéries anaérobies qui vivent dans le rumen.

Il y a alors production de différents acides gras volatils (AGV) (acétate, propionate et butyrate (par ordre d'importance)), de gaz carbonique ($CO_2$) et d'hydrogène ($H_2$).

Le rumen étant un milieu anaérobie, la production d'énergie (ATP) se fait par « transfert d'hydrogène »

L'hydrogène inhibe l'activité de la plupart des bactéries par différents mécanismes. L'hydrogène produit doit donc être éliminé du rumen pour favoriser une bonne digestion microbienne.

La méthanogenèse, aboutissant à la formation de méthane ($CH_4$) est la principale voie permettant cette élimination. Elle peut être symbolisée par la réaction suivante :

$$[CO_2 + 4\ H_2] = CH_4 + 2H_2O$$

Cette transformation est assurée par des bactéries méthanogènes qui vivent associées aux protozoaires (qui constituent la microfaune ruminale).

Le méthane ($CH_4$) est, avec le dioxyde de carbone ($CO_2$) le protoxyde d'azote ($N_2O$) et les trois carbures halogénés (chlorofluorocarbures CFC, hydrofluorocarbures HFC, perfluorocarbures PFC), l'un des principaux gaz à effet de serre (G ES).

Sa contribution à l'effet de serre est très importante. En effet, une molécule de $CH_4$ équivaut à 21 molécules de $CO_2$. selon les tableaux d'équivalence officiels.

Ces GES sont mis en cause dans les changements climatiques, notamment dans le réchauffement climatique. Lutter contre ces changements est devenu, depuis le Sommet de la Terre, à Rio en 1992, un engagement international qui s'est traduit en 1997 par des engagements quantitatifs, lesquels ont été repris au travers du protocole de Kyoto.

Le Conseil Européen s'est ainsi fixé l'objectif de réduire de 20% les émissions de GES d'ici 2020. En 2003, l'élevage engendrait 47,7 $MteqCO_2$ (millions de tonnes équivalent $CO_2$), dont 28,3 $MteqCO_2$ sous forme de méthane provenant des fermentations digestives des ruminants (Leseur, 2006 les marchés du carbone: quelle place pour l'agriculture française (Agriculture de france)" ; Martin et al., 2006 Methane output and diet digestibility in response to feading clairy cows calde linseed extraded baseed, or linseed oil" (J. Anim Science).

Ces émissions de méthane représentent 26% des émissions du secteur agricole et 5% des émissions françaises de GES (Leseur, 2006).

Il apparaît donc nécessaire de réduire les émissions de méthane par les ruminants.

Différentes solutions ont été proposées pour atteindre cet objectif.

La première consiste à limiter la consommation de produits issus de ruminants et notamment de produits laitiers, ce qui aurait pour effet de diminuer le nombre de ces ruminants laitiers, et donc fort logiquement les émissions de méthane.

Mais les produits laitiers sont présents dans l'alimentation des êtres humains depuis la nuit des temps. L'homme est connu pour être la seule espèce animale à consommer des produits laitiers après le sevrage. De plus, au plan nutritionnel, ces produits laitiers contiennent du calcium, des protéines et des lipides aux propriétés nutritionnelles très particulières, indissociables d'une alimentation équilibrée, à tous les stades de la vie.

Par ailleurs, l'augmentation de la population sur la planète semble difficilement compatible avec une réduction des effectifs d'animaux destinés à nourrir cette population en augmentation.

Une autre solution consiste à modifier l'alimentation des ruminants laitiers (vaches, brebis, chèvres, etc...) en orientant les mécanismes de la rumination de façon à produire moins de méthane. L'article- B. VLAEMINCK, V. FIEVEZ: "Milk odd and branched-chain fatty acids to predict ruminal methanogenesis in dairy cows", COMMUNICATIONS IN AGRICULTURAL AND APPLIED BIOLOGICAL SCIENCES, vol. 70, no. 2, 2005, pages 43-47

divulgue un procédé d'évaluation de la quantité de méthane produite par un ruminant laitier, basé sur un modèle mathématique ayant pour paramètres les teneurs en acides gras et en lactose du lait . D'autre part, un second modèle propose d'établir une relation entre la quantité de méthane produite par un ruminant laitier la proportion d'acides gras, à savoir C15:0 et iso C15:0.

Différentes techniques ont été proposées pour atteindre cet objectif.

La première consiste à augmenter la quantité de lait produit par vache (intensification de l'élevage).

La deuxième prévoit de donner aux vaches des additifs toxiques pour les protozoaires et/ou pour les bactéries méthanogènes de façon à produire du lait en réduisant les quantités émises de méthane.

Enfin, une troisième technique consiste à introduire dans l'alimentation des ruminants laitiers des sources de lipides végétaux riches en acides gras insaturés, préférentiellement de la famille Oméga 3 ou sous forme d'autres acides gras insaturés bien que leurs effets sur la méthanogénèse soient moins puissants (que ceux des acides gras Oméga 3). Ces acides gras sont toxiques pour les bactéries méthanogènes, directement ou via des effets toxiques sur les protozoaires qui vivent associés à ces bactéries méthanogènes.

Mais ces différentes techniques ne sauraient présenter un véritable intérêt que si l'on disposait d'un procédé pratique et facile à mettre en oeuvre de la quantité de méthane produite par les ruminants.

Or, une mesure des émissions de méthane n'est possible à ce jour que dans des stations expérimentales, et ceci de façon coûteuse et lourde.

La présente invention vise à combler cette lacune.

Ainsi, selon un premier aspect, la présente invention concerne un procédé d'évaluation de la quantité de méthane produite par un ruminant laitier, caractérisé par le fait qu'il comprend la détermination du rapport entre la quantité d'acides gras avec 16 atomes de carbone ou moins, dite AG<C16 et la somme des acides gras totaux du lait, lesdits acides gras étant ceux présents dans le lait produit par ledit ruminant, ladite quantité de méthane étant définie par la relation suivante :

$$\text{Quantité } CH_4 = (AG\!<\!C16/AG\ totaux) * a * (\text{production de lait})^b$$

où

quantité $CH_4$ (en g/litre de lait) = quantité de méthane produite ; AG<C16 = quantité d'acides gras avec 16 atomes de carbone ou moins ; AG totaux = quantité totale d'acides gras ;

expression du rapport (AG<C16)/(AG totaux) en % des AG totaux ; production de lait = quantité de lait produite en kg de lait/animal et par an ;

a et b sont des paramètres numériques, avec a compris entre 10 et 13 et b compris entre - 0,40 et - 0,45.

Ce procédé est facile à mettre en oeuvre puisque le présent demandeur a mis en avant le fait que la quantité de méthane produite est directement liée à la quantité d'acides gras dans le lait. Or, le dosage des acides gras du lait est une opération très largement mise en oeuvre actuellement, qui ne requiert pas de moyens sophistiqués et/ou coûteux.

Dans un mode de réalisation préféré, a et b valent respectivement 11.368 et -0,4274.

Un autre aspect de l'invention se rapporte à un procédé pour diminuer et contrôler la quantité de méthane produite par un ruminant laitier.

Ce procédé est remarquable en ce qu'il consiste :

- à fournir au ruminant une ration alimentaire qui répond à au moins l'un des critères suivants :

    a) elle exclut toute matière grasse d'origine animale ;
    b) elle limite l'apport exogène d'huile végétale contenant plus de 30% des AG totaux sous forme d'AG saturés, en l'état, hydrogénée ou saponifiée, à au plus 15 grammes / animal et par 100 kg de poids vif ;
    c) elle contient au moins une source lipidique riche en acide alpha-linolénique oméga 3 (ALA), c'est-à-dire dont plus de 30 % des AG totaux est sous forme d'AG oméga 3 ;

    et à contrôler ladite quantité de méthane en mettant en oeuvre le procédé selon l'une des caractéristiques précédentes.

Préférentiellement, cette source lipidique se présente sous forme de fourrage pâturé ou conservé (fané, ensilé, enrubanné, déshydraté...) ou de graines oléagineuses (graines en l'état, crues ou cuites) riches en ALA ou de tourteaux gras de ces mêmes graines oléagineuses.

Avantageusement, ladite source inclut du lin.

D'autres caractéristiques et avantages de l'invention apparaitront de la description détaillée qui va suivre.

1- <u>Production d'acides gras volatils (AGV) et production de méthane ($CH_4$)</u>

Le lien entre la production des AGV dans le rumen et la production de méthane est connu et étudié depuis de nombreuses années.

Ainsi, il a été montré que la production d'acétate et de butyrate libère de l'hydrogène et favorise donc la production de méthane, alors que la production de propionate permet l'utilisation de l'hydrogène et donc limite la production de méthane (Gworgwor et al., 2006 "Environnemental Implication of Methane production by Ruminents: A review" (Journal of Sustainable Development in Agriculture and Environnement).

Cela peut être illustré par les relations suivantes :

1 Glucose (C6) donne 2 Pyruvates (C3) [+ 4H]
1 Pyruvate (C3) + $H_2O$ = 1 Acétate + $CO_2$ [+ 2 H] et
1 Pyruvate = 1 propionate (C3) [- 4 H]

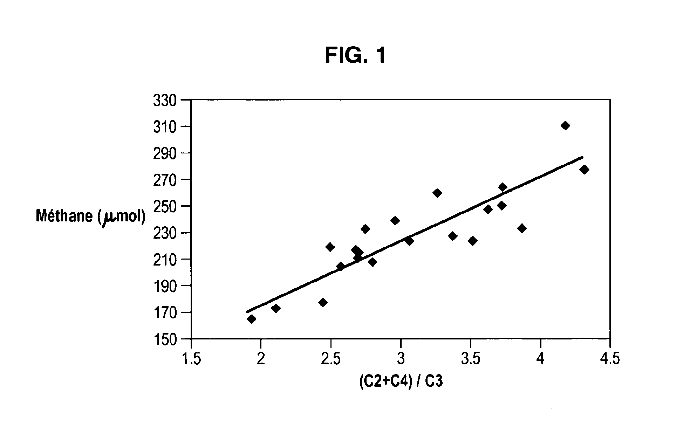

Une équation de prédiction a donc été mise au point pour prédire la production de $CH_4$ à partir de la production d'AGV, grâce au graphique illustré à la figure 1 annexée (Moss et al., 2000). Ainsi, plus les fermentations du rumen produisent de C2 et de C4, et plus la production de $CH_4$ est importante.

A l'inverse, plus les fermentations du rumen produisent de C3, et plus la production de $CH_4$ est diminuée.

L'équation de synthèse qui en découle est définie comme suit :

$$[CH4] = 0,45 \, [acétate] + 0,40 \, [Butyrate] - 0,275 \, [Propionate]$$

où [x] = quantité de x, en % des AGV totaux

2- Influence de l'apport d'une source (digestible dans le rumen) d'Acide alpha-linolénigue Omega 3 (ALA) sur la production d'AGV et de CH4

L'acide alpha-linolénique Oméga 3 ou C18 :3 n-3 ou « ALA » est un constituant de masse des végétaux en croissance.

On en trouve par exemple de grandes quantités dans l'herbe jeune et dans les algues où il constitue la grande majorité (de 50 à 75%) des acides gras de ces plantes. En effet, l'ALA est un constituant de base des membranes chlorophylliennes.

L'ALA est également présent dans certaines graines oléagineuses comme la graine de lin (de 45 à 70%), de chanvre (environ 15%), de colza ou de soja (environ 10%)

L'apport d'ALA dans la ration des ruminants laitier modifie la population microbienne présente dans le rumen. Effectivement, l'ALA inhibe directement et indirectement les bactéries méthanogènes et modifie significativement les proportions d'AGV produits en baissant notamment les quantités d'acétate et de butyrate produites.

Selon de nombreuses sources de la littérature scientifique, lorsqu'une source d'ALA est ajoutée dans la ration d'un ruminant, la production d'acide propionique (C3) augmente, et les proportions d'acides acétique (C2) et butyrique (C4) diminuent.

Il apparaît donc que :

- le rapport [(C2+C4) sur C3] est un très bon indice de la production de méthane dans le rumen ;
- l'apport d'ALA dans les régimes des ruminants laitiers a un effet linéaire sur le rapport (C2+C4)/C3 qui diminue régulièrement lorsque, toutes choses égales par ailleurs, la quantité d'ALA ingérée par le ruminant laitier augmente.

On notera toutefois que les sources d'ALA peuvent avoir des effets différents selon le lieu de digestion de cet ALA.

Ainsi, les graines de lin crues ne réduiront que faiblement le rapport [(C2+C4)/C3)], alors que les graines de lin extrudées et les huiles de lin le réduiront fortement.

L'aptitude à modifier ce rapport [(C2+C4)/C3)] est donc liée à la quantité d'ALA dans le régime des animaux, mais aussi à la disponibilité de cet ALA dans le rumen.

3- Influence du ratio [(C2+C4)/C3)] sur la composition du lait

Les AGV (C2, C3, ou C4) produits par le rumen diffusent au travers des parois de celui-ci, ou passent plus loin la barrière intestinale et se retrouvent dans les liquides circulants.

L'acide propionique (C3) est utilisé comme « glucoformateur » et contribue à la production laitière comme précurseur du lactose.

Au contraire, les acides acétique (C2) et butyrique (C4) sont utilisés par les mécanismes de synthèse de novo pour produire les acides gras saturés de 2 à 16 atomes de carbone du lait.

En effet, cette synthèse, qui se produit dans les cellules épithéliales mammaires, utilise l'acetyl coA, un composé issu de C2 et/ou C4 pour ces synthèses de C14 :0 et de C16 :0.

Ces 2 acides gras sont ensuite « raccourcis » (beta-oxydation peroxisomale) pour produire les acides gras à chaîne courte et moyenne du lait. Ces acides gras peuvent ensuite être éventuellement désaturés en acides gras mono-insaturés par l'action d'une désaturase mammaire.

4- Modèle théorique

La quantité de $CH_4$ produite par litre de lait tient donc compte de :

a) la production laitière par an de l'animal.
Plus une vache laitière (par exemple) produit de lait, et plus la production de méthane par litre de lait diminue. Ainsi, certains auteurs proposent l'équation suivante :

Quantité de méthane produite (en kg par vache et par an) = 55,7 + 0,0098 * (production laitière , en kg par an et par animal).

b) la composition de la ration et notamment la quantité d'ALA disponible dans le rumen.
c) le ratio (C2+C4)/C3 dans le rumen de ces animaux. Ce ratio d'AGV se lit avec une causalité biologique forte dans la composition du lait, sous la forme du ratio entre :

i. la somme des AG du lait à 16 ou moins de 16 atomes de Carbone
et
ii. la somme de tous les AG du lait

La quantité de $CH_4$ produite par femelle laitière peut donc se calculer en fonction de la production laitière (en kg de lait par an par animal) et de la composition du lait en AG de cet animal.

Il apparaît donc que l'homme de l'art peut ainsi disposer d'un outil précis pour évaluer la production de méthane des animaux laitiers, en fonction de leur niveau de production (facilement mesurable) et de la composition du lait (facilement mesurable).

Cette mesure indirecte mais précise de la production de méthane pourra permettre d'orienter les systèmes de rationnement des animaux ruminants laitiers, de façon à réduire leur contribution à l'effet de serre et permettra de mesurer rapidement les effets de ces modifications.

5- Essais et interprétation des résultats

De nombreux essais sont disponibles dans la bibliographie générale, qui décrivent les effets d'un apport d'une source d'ALA alimentaire (sous forme de lin le plus souvent) sur la production de méthane des vaches, chèvres ou autres femelles de ruminant laitier

D'autres essais sont disponibles, qui décrivent les effets de ces mêmes sources d'ALA sous forme de lin sur la composition en acides gras (AG) du lait.

Le rapprochement et la synthèse de ces résultats a été mis en oeuvre pour valider le modèle théorique. Ces résultats sont présentés dans le Tableau 1 ci-après.

On s'est ici intéressé à la mesure de la production de $CH_4$ par litre de lait en fonction :

- de la production de lait (kg / animal /an) ;
- du rapport entre les AG du lait avec 16 atomes de carbone ou moins en % des AG totaux.

Nous aurions bien entendu pu choisir d'autres AG ou d'autres sommes ou ratio d'AG pour illustrer les effets de la synthèse de-novo d'AG saturés dans la mamelle, à partir du C2 produit dans le rumen avec émission de CH4.

Toutefois, la somme des AG saturés avec 16 ou moins d'atomes de carbone est particulièrement représentative de cette synthèse de-novo à partir du C2. Mais la lecture des acides gras en C16 ou de la somme des AG à 12, 14, 16 atomes de C, voire le ratio entre C16 et la somme des AG saturés seraient également des critères pertinents.

Cette compilation de résultats a été réalisée à partir notamment d'essais présentant d'une part des profils d'AG de lait, d'autre part de mesures de méthane produit par litre de lait dans des essais utilisant comme source d'ALA des graines de lin extrudées, avec des vaches à différents niveaux de production.

Tableau 1 Production de méthane par litre de lait en fonction de la production laitière par animal et du profil d'AG du lait dans une plage donnée de profil d'AG.du lait

| AG / PL | 4000 | 5000 | 6000 | 7000 | 8000 | 9000 | 10000 | 11000 | 12000 | 13000 | 14000 |
|---|---|---|---|---|---|---|---|---|---|---|---|
| 70 | 23,7 | 20,9 | 19,1 | 17,8 | 16,8 | 16,0 | 15,4 | 14,9 | 14,4 | 14,1 | 13,8 |
| 68 | 23,0 | 20,3 | 18,5 | 17,2 | 16,3 | 15,5 | 14.9 | 14,4 | 14,0 | 13,7 | 13,4 |
| 66 | 22,4 | 19,7 | 18,0 | 16,7 | 15,8 | 15,1 | 14,5 | 14,0 | 13,6 | 13,3 | 13,0 |
| 64 | 21,7 | 19,1 | 17,4 | 16,2 | 15,3 | 14.6 | 14,1 | 13,6 | 13,2 | 12,9 | 12,6 |
| 62 | 21,0 | 18,5 | 16,9 | 15,7 | 14,8 | 14,2 | 13,6 | 13,2 | 12,8 | 12,5 | 12,2 |
| 60 | 20,3 | 17,9 | 16,4 | 15,2 | 14,4 | 13,7 | 13,2 | 12,7 | 12,4 | 12,1 | 11,8 |
| 58 | 19,7 | 17.4 | 15,8 | 14,7 | 13,9 | 13,2 | 12,7 | 12,3 | 12,0 | 11,7 | 11,4 |
| 56 | 19,0 | 16,8 | 15,3 | 14,2 | 13,4 | 12,8 | 12,3 | 11,9 | 11,6 | 11,3 | 11,0 |
| 54 | 18,3 | 16,2 | 14,7 | 13,7 | 12,9 | 12,3 | 11,9 | 11,5 | 11,1 | 10,9 | 10,6 |
| 52 | 17,6 | 15,6 | 14,2 | 13,2 | 12,5 | 11,9 | 11,4 | 11,0 | 10,7 | 10,5 | 10,2 |
| 50 | 16,9 | 15,0 | 13,6 | 12,7 | 12,0 | 11,4 | 11,0 | 10,6 | 10,3 | 10,1 | 9,8 |
| 48 | 16,3 | 14,4 | 13,1 | 12,2 | 11,5 | 11,0 | 10,5 | 10,2 | 9,9 | 9,7 | 9,4 |
| 46 | 15,6 | 13,8 | 12,5 | 11,7 | 11,0 | 10,5 | 10,1 | 9,8 | 9,5 | 9,3 | 9,1 |
| 44 | 14,9 | 13,2 | 12,0 | 11,2 | 10,5 | 10,1 | 9,7 | 9,3 | 9,1 | 8,9 | 8,7 |
| 42 | 14,2 | 12,6 | 11,5 | 10,7 | 10,1 | 9,6 | 9,2 | 8.9 | 8,7 | 8,5 | 8,3 |

NB : Volontairement, cette plage de profil d'AG du lait a été limitée à la plage où les AG avec 16 ou moins atomes de carbone constituent de 70 à 42 % des AG du lait. Non que le modèle biologique ou que l'équation qui en découle soit limitée à cette plage, mais parce que au-delà des limites de cette plage, la composition des laits ainsi produits peut être considérée comme d'une part nutritionnellement douteuse, et d'autre part incompatible avec les moyens mis en oeuvre pour obtenir ces laits et définis plus haut.

Dans ce tableau : PL (horizontalement) = production de lait (Kg/animal/an) et AG = [AG<C16/AG totaux], en %.

Nous avons donc établi un tableau de prédiction des valeurs de CH4 émises à partir des données de production de lait et des profils d'AG de ces laits ainsi produits :

Tableau 2

| AG / PL | 4000 | 5000 | 6000 | 7000 | 8000 | 9000 | 10000 | 11000 | 12000 | 13000 | 14000 |
|---|---|---|---|---|---|---|---|---|---|---|---|
| 70 | 23,0 | 20,9 | 19,3 | 18,1 | 17,1 | 16,2 | 15,5 | 14,9 | 14,4 | 13,9 | 13,5 |
| 68 | 22,3 | 20,3 | 18,8 | 17,6 | 16,6 | 15,8 | 15,1 | 14,5 | 14,0 | 13,5 | 13,1 |
| 66 | 21,7 | 19,7 | 18,2 | 17,1 | 16,1 | 15,3 | 14,6 | 14,1 | 13,5 | 13,1 | 12,7 |
| 64 | 21,0 | 19,1 | 17,7 | 16,5 | 15,6 | 14,9 | 14,2 | 13,6 | 13,1 | 12,7 | 12,3 |
| 62 | 20,3 | 18,5 | 17,1 | 16,0 | 15,1 | 14,4 | 13,8 | 13,2 | 12,7 | 12,3 | 11,9 |
| 60 | 19,7 | 17,9 | 16,6 | 15,5 | 14,6 | 13,9 | 13,3 | 12,8 | 12,3 | 11,9 | 11,5 |
| 58 | 19,0 | 17,3 | 16,0 | 15,0 | 14,2 | 13,5 | 12,9 | 12,4 | 11,9 | 11,5 | 11,1 |
| 56 | 18,4 | 16,7 | 15,5 | 14,5 | 13,7 | 13,0 | 12,4 | 11,9 | 11,5 | 11,1 | 10,8 |
| 54 | 17,7 | 16,1 | 14,9 | 14,0 | 13,2 | 12,5 | 12,0 | 11,5 | 11,1 | 10,7 | 10,4 |
| 52 | 17,1 | 15,5 | 14,4 | 13,4 | 12,7 | 12,1 | 11,5 | 11,1 | 10,7 | 10,3 | 10,0 |
| 50 | 16,4 | 14,9 | 13,8 | 12,9 | 12,2 | 11,6 | 11,1 | 10,7 | 10,3 | 9,9 | 9,6 |
| 48 | 15,8 | 14,3 | 13,2 | 12,4 | 11,7 | 11,1 | 10,6 | 10,2 | 9,9 | 9,5 | 9,2 |

(suite)

| AG / PL | 4000 | 5000 | 6000 | 7000 | 8000 | 9000 | 10000 | 11000 | 12000 | 13000 | 14000 |
|---|---|---|---|---|---|---|---|---|---|---|---|
| 46 | 15,1 | 13,7 | 12,7 | 11,9 | 11,2 | 10,7 | 10,2 | 9,8 | 9,4 | 9,1 | 8,8 |
| 44 | 14,4 | 13,1 | 12,1 | 11,4 | 10,7 | 10,2 | 9,8 | 9,4 | 9,0 | 8,7 | 8,5 |
| 42 | 13,8 | 12,5 | 11,6 | 10,9 | 10,3 | 9,7 | 9,3 | 8,9 | 8,6 | 8,3 | 8,1 |

6- <u>Validation de ce modèle</u>

Nous disposons des résultats de plusieurs essais qui ont mesuré précisément :

- la quantité d'ALA (disponible dans le rumen) dans les rations de vaches laitières ;
- les effets de cet ALA sur le ratio (C2+C4) / C3 ;
- les effets de ces rations sur le profil d'AG du lait ;
- et enfin d'essais qui ont la production de CH4 par litre de lait avec différentes teneurs en ALA des rations.

Le tableau ci-dessous compare les valeurs mesurées aux valeurs "prédites" à partir des tableaux précités.

Tableau 3

| Essais | 1 | 2 | 3 | 4 | 5 | 6 |
|---|---|---|---|---|---|---|
| | | | | | | |
| Prod lait | 6000 | 6000 | 7000 | 7000 | 7000 | 6000 |
| AG >=C16 | 61 | 42 | 66 | 64 | 56 | 42 |
| | | | | | | |
| CH4 en g/l lait | 16,8 | 11,8 | 17,2 | 16,2 | 13,8 | 10,9 |
| | | | | | | |
| "Prédiction" en g/l | | | | | | |
| Selon tableau 1 | 16,4 | 11,5 | 17,2 | 16,2 | 14,2 | 11,5 |
| Selon tableau 2 | 16,4 | 11,5 | 17,2 | 15,7 | 14,2 | 11,5 |

Le présent demandeur a déduit de ces essais, que la quantité de méthane peut être définie par la relation suivante :

$$\text{Quantité } CH_4 = (AG{<}C16/AG\ totaux) * a * (\text{production de lait})^b$$
où

quantité $CH_4$ (en g/litre de lait) = quantité de méthane produite ;
AG<C16 = quantité d'acides gras avec 16 atomes de carbone ou moins ;
AG totaux = quantité totale d'acides gras ;
production de lait = quantité de lait produite en kg par animal et par an
a et b = paramètres numériques, avec a compris entre 10 et 13, et b compris entre - 0,40 et - 0,45.
Préférentiellement, les paramètres a et b valent respectivement 11,368 et -0,4274.

**Revendications**

1. Procédé d'évaluation de la quantité de méthane produite par un ruminant laitier, **caractérisé par le fait qu'**il comprend la détermination du rapport entre la quantité d'acides gras avec 16 atomes de carbone ou moins, dite AG<C16 et la somme des acides gras totaux du lait, lesdits acides gras étant ceux présents dans le lait produit par ledit ruminant, ladite quantité de méthane étant définie par la relation suivante :

$$\text{Quantité CH}_4 = (\text{AG<C16/AG totaux}) * a * (\text{production de lait})^b$$

où
quantité *CH$_4$ (en g/litre de lait) = quantité de méthane produite ;
AG<C16 = quantité d'acides gras avec 16 atomes de carbone ou moins ;
AG totaux = quantité totale d'acides gras ;
expression du rapport (AG<C16)/(AG totaux) en % des AG totaux ;
production de lait = quantité de lait produite en kg de lait/animal et par an ;
a et b sont des paramètres numériques, avec a compris entre 10 et 13 et b compris entre - 0,40 et - 0,45.

2. Procédé selon la revendication précédente, **caractérisé par le fait que** a et b valent respectivement 11.368 et -0,4274.

3. Procédé pour diminuer et contrôler la quantité de méthane produite par un ruminant laitier, **caractérisé par le fait qu'**il consiste à lui fournir une ration alimentaire qui répond à au moins l'un des critères suivants :

   a) elle exclut toute matière grasse d'origine animale ;
   b) elle limite l'apport exogène d'huile végétale contenant plus de 30% d'AG saturés, en l'état, saponifiée ou hydrogénée, à au plus 15 grammes / animal et par 100 kg de poids vif ;
   c) elle contient au moins une source lipidique riche en acide alpha-linolénique oméga 3 (ALA), c'est-à-dire dont plus de 30 % des AG totaux est sous forme d'AG oméga 3 ;
   et à contrôler ladite quantité en mettant en oeuvre le procédé selon l'une des revendications 1 ou 2.

4. Procédé selon la revendication 3, **caractérisé par le fait que** l'on fournit une ration qui répond cumulativement auxdits trois critères.

5. Procédé selon l'une des revendications 3 ou 4, **caractérisé par le fait que** l'on utilise une source lipidique riche en ALA sous forme de fourrage pâturé ou conservé, notamment fané, ensilé, enrubanné, ou déshydraté, ou de graines oléagineuses notamment de graines en l'état, crues ou cuites, riches en ALA ou de tourteaux gras de ces mêmes graines oléagineuses.

6. Procédé selon la revendication précédente, **caractérisé par le fait que** ladite source inclut du lin.

**Claims**

1. Method to evaluate the quantity of methane produced by a dairy ruminant, **characterized by** the fact that it comprises determining the ratio between the quantity of fatty acids with 16 carbon atoms or less, called FA<C16 and the sum of the total fatty acids in the milk, said fatty acids being those found in the milk produced by said ruminant, said quantity of methane being defined by the following equation:

$$\texttt{Quantity CH}_4 = (\texttt{FA<C16/Total FAs})*a*(\texttt{milk production})^b$$

in which:
quantity CH$_4$ (in g/litre of milk) = quantity of methane produced;
FA<C16 = quantity of fatty acids with 16 carbon atoms or less;
Total FAs = total quantity of fatty acids;
expression of the ratio (FA<C16) / (Total FAs) as a % of total FAs;
milk production = quantity of milk produced in kg of milk/animal and per year;
a and b are numerical parameters, with a lying between 10 and 13, and b lying between -0.40 and -0.45.

2. Method according to the preceding claim, **characterized by** the fact that a and b are respectively 11.368 and -0.4274.

3. Method to reduce and control the quantity of methane produced by a dairy ruminant, **characterized by** the fact that it consists of giving it a food ration which meets at least one of the following criteria:

a) it excludes all fat of animal origin;

b) it limits the exogenous intake of vegetable oil containing more than 30% saturated FAs, in the natural state, saponified, or hydrogenated, to no more than 15 grams/animal and per 100 kg live weight;

c) it contains at least one lipid source high in Omega-3-alpha-linolenic acid (ALA) i.e. of which over 30% of the Total FAs is in the form of Omega-3 FAs;

and of controlling said quantity by applying the method according to either of claims 1 or 2.

**4.** Method according to claim 3, **characterized by** the fact that a ration is given which accumulatively meets said three criteria.

**5.** Method according to claim 3, **characterized by** the fact that a lipid source high in ALA is used in the form of grazed fodder or conserved forage, in particular wilted grass, silage, wrapped round bale or dehydrated, or oilseeds notably oilseeds in the natural state, raw or cooked high in ALA, or oilcakes of these same oilseeds.

**6.** Method according to the preceding claim, **characterized by** the fact that said source includes flax.


**Patentansprüche**

**1.** Verfahren zur Ermittlung der durch einen milchgebenden Wiederkäuer erzeugten Methanmenge, **dadurch gekennzeichnet, dass** es die Bestimmung des Verhältnisses der Menge an Fettsäuren mit 16 oder weniger Kohlenstoffatomen, bezeichnet als AG<C16, zu der Summe der Gesamtfettsäuren der Milch umfasst, wobei die Fettsäuren diejenigen sind, die in der von dem Wiederkäuer produzierten Milch vorliegen, wobei die Methanmenge durch folgende Gleichung bestimmt wird:

$$\text{CH4-Menge} = (\text{AG<C16}/\text{AG gesamt}) * a * (\text{Milchproduktion})^b$$

worin

CH4-Menge (in g/Liter Milch) = Menge des erzeugten Methans;

AG<C16 = Menge der Fettsäuren mit 16 oder weniger Kohlenstoffatomen;

AG gesamt = Gesamtmenge an Fettsäuren;

Ausdruck des Verhältnisses (AG<C16)/(AG gesamt) in % der Gesamtfettsäuren;

Milchproduktion = Menge der produzierten Milch in kg Milch/Tier und pro Jahr;

a und b numerische Parameter sind, wobei a 10 bis 13 beträgt und b -0,40 bis -0,45 beträgt.

**2.** Verfahren gemäß dem vorherigen Anspruch, **dadurch gekennzeichnet, dass** a und b einen Wert von 11,368 bzw. -0,4274 haben.

**3.** Verfahren zur Verminderung und Regulierung der durch einen milchgebenden Wiederkäuer erzeugten Methanmenge, **dadurch gekennzeichnet, dass** das Verfahren darin besteht, dem Wiederkäuer eine Nahrungsration zu verabreichen, die wenigstens eines der folgenden Kriterien erfüllt:

a) sie schließt sämtliche Fette tierischen Ursprungs aus;

b) sie begrenzt das exogene Angebot an pflanzlichem Öl, welches mehr als 30% gesättigte Fettsäuren im verseiften oder hydrierten Zustand enthält, auf höchstens 15 Gramm/Tier und pro 100 kg Lebendgewicht;

c) sie enthält wenigstens eine Lipidquelle, welche reich an Omega-3-Alpha-Linolensäure (ALA) ist, d. h., bei der mehr als 30 % der Gesamtfettsäuren in Form von Omega-3-Fettsäure vorliegen,

und die erzeugte Methanmenge durch Anwendung des Verfahrens gemäß einem der Ansprüche 1 oder 2 zu regulieren.

**4.** Verfahren gemäß Anspruch 3, **dadurch gekennzeichnet, dass** man eine Ration verabreicht, die gleichzeitig alle drei Kriterien erfüllt.

**5.** Verfahren gemäß einem der Ansprüche 3 oder 4, **dadurch gekennzeichnet, dass** man eine Lipidquelle verwendet, die reich an ALA ist, in Form von abgeweidetem oder konserviertem Viehfutter, insbesondere Heu, gewickelt oder getrocknet, oder Ölsamen, insbesondere Samen in rohem oder gekochtem Zustand, welche reich an ALA sind, oder Fettkuchen dieser Ölsamen.

**6.** Verfahren gemäß dem vorherigen Anspruch, **dadurch gekennzeichnet, dass** die Quelle Lein(samen) enthält.

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **B. VLAEMINCK ; V. FIEVEZ.** Milk odd and branched-chain fatty acids to predict ruminal methanogenesis in dairy cows. *COMMUNICATIONS IN AGRICULTURAL AND APPLIED BIOLOGICAL SCIENCES,* 2005, vol. 70 (2), 43-47

- **GWORGWOR et al.** Environnemental Implication of Methane production by Ruminents: A review. *Journal of Sustainable Development in Agriculture and Environnement,* 2006